# EUROPEAN PATENT SPECIFICATION

(11) **EP 3 835 803 B1**
(45) Date of publication and mention of the grant of the patent: **27.03.2024**
(21) Application number: 19215964.8
(22) Date of filing: 13.12.2019
(51) Int. Cl.: G01R 33/58, G01R 33/54, G01R 33/48, G06N 3/08

(54) **SYSTEM AND METHOD FOR ESTIMATING A RELATIVE SUBSTANCE COMPOSITION OF A PORTION OF A BODY OF A PATIENT**
SYSTEM UND VERFAHREN ZUR SCHÄTZUNG EINER RELATIVEN SUBSTANZZUSAMMENSETZUNG EINES TEILS EINES KÖRPERS EINES PATIENTEN
SYSTÈME ET PROCÉDÉ D'ESTIMATION D'UNE COMPOSITION DE SUBSTANCE RELATIVE D'UNE PARTIE DU CORPS D'UN PATIENT

(43) Date of publication of application: 16.06.2021
(73) Proprietor: Siemens Healthineers AG, 91301 Forchheim (DE)
(72) Inventor: Sukkau, Johann, 91074 Herzogenaurach (DE)
(74) Representative: Siemens Healthineers Patent Attorneys

(56) References cited:
- WO-A1-2014/082128
- US-A1- 2013 249 552
- US-A1- 2015 168 521

## Description

The present invention relates to a system and a method for estimating a relative substance composition, RSC, of a portion of a patient's body. Moreover, the invention relates to a method for training a machine learning algorithm for use in such a system and such a method as well as to a method for generating training data for such a training method.

In magnetic resonance, MR, scans (i.e. image acquisitions), the nuclear spins of hydrogen atoms are resonantly excited by radio frequency pulses. The resonance frequency of each spin is proportional to the magnetic field B0 at the location of the nucleus of the hydrogen atoms. Therefore, the terms "frequency" and "B0" are sometimes used as synonyms.

US 2015/168521 A1 discloses a method for determining a reception gain for receiving magnetic resonance signals of an object.

In any MR scan, the local B0 field can vary for a number of reasons. For example, the magnetic field strength can drift, e.g. due to temperature changes in the magnet cooling shields which can lead to global frequency shifts. Also, the susceptibility of biological matter in the patient's body and its boundaries can lead to local frequency shifts, generally addressed by shimming. Shimming is a process by which the main magnetic field is made more homogeneous.

A shimming method is described for example in US 2015/00771155 A1, in which it is proposed to acquire magnetic resonance data by measurements implemented at two different echo times whose difference forms a dephasing time after an excitation at at least two different dephasing times.

Microscopic frequency shifts can also be caused as a result of the chemical bonds of the hydrogen atom, for example in fat or water molecules. The chemical shifts are usually determined in parts per million, ppm, as they depend on the magnetic field strength applied.

In order to achieve best possible contrast and signal-to-noise-ratio, SNR, and to successfully employ techniques such as fat or water saturation, the resonance frequencies of the hydrogen atoms within the field of view of each MR scan have to be precisely known and used for setting an MR device up for a scan. Therefore, usually a frequency adjustment measurement is conducted for each MR scan using a specialized sequence. The result is a spectrum of signal intensities per frequency inside the field of view of the MR scanning device. These spectra can be designated as resonance frequency spectra, RFS, which typically show distinct peaks generated by resonance frequency maxima of different substances (such as water, fat, silicone, ...) within a patient's body.

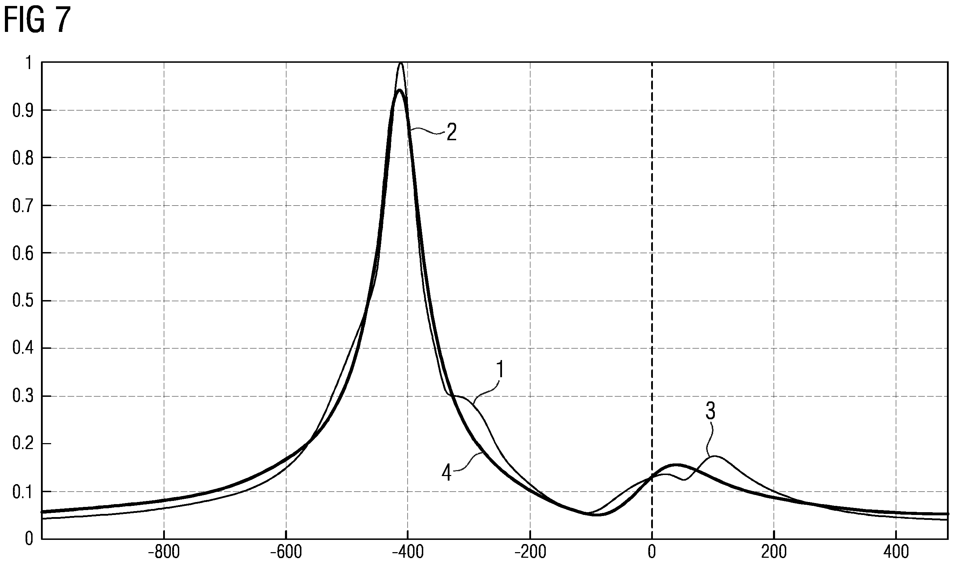

These resonance frequency spectra usually comprise peaks that correspond to resonance frequency maxima for different substances such as fat or water. Fig. 7 shows a schematic resonance frequency spectrum 1 with a distinct peak 2 for the resonance frequency of (body) fat and a distinct second peak 3 for the resonance frequency of water. The horizontal axis shows frequency values as deviations from a currently set frequency value for the resonance frequency of water, and the vertical axis signal strength in arbitrary units. Current algorithms essentially determine the peaks (for water: dashed line) by adjusting Lorentzian functions to the resonance frequency spectrum, RFS, and use an optimization to create a best fit. In this way, a fitting curve 4 for a resonance frequency spectrum, RFS, as shown in Fig. 7 is usually generated automatically.

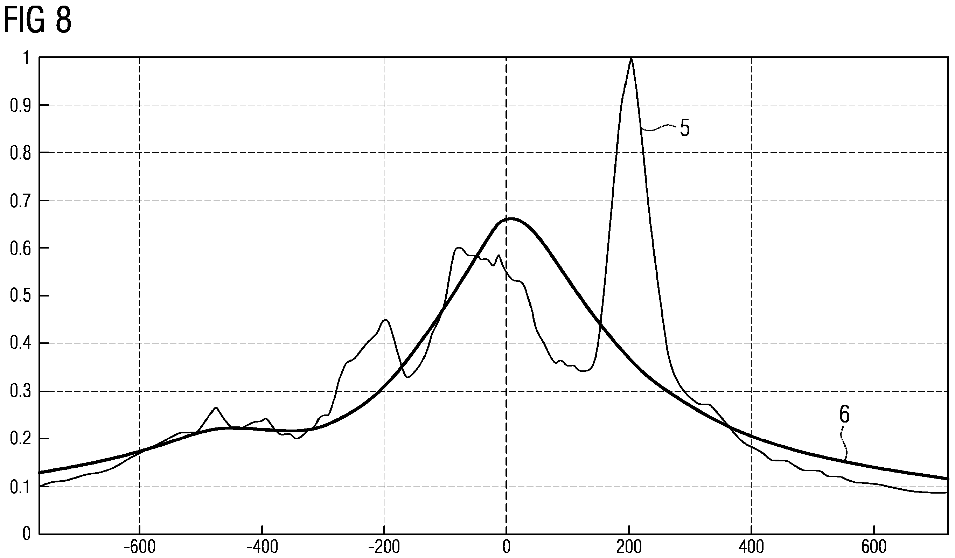

However, in other cases, as illustrated in Fig. 8, the measured resonance frequency spectrum 5 can be more complicated so that the fitting curve 6 generated by the prior art algorithms often fails to capture the details of the resonance frequency spectrum, RFS.

In each resonance frequency spectrum, RFS, at least the "water peak" (i.e. the peak resulting from the resonance frequency of hydrogen atoms bound in water molecules) has to be identified. Automated systems, however, are struggling to identify the correct peaks within such RFS with the corresponding substance as their relative positions and heights vary, in particular due to the different substance composition ratios, SCRs, within each particular body and the field of view chosen for a specific scan thereof. If two peaks are confused, for example if the "fat peak" (i.e. the peak resulting from the resonance frequency of hydrogen atoms bound in fat molecules) is confused for the "water peak", the water resonance frequency setting of the MR device may be mistaken by 3.4ppm. As a result, fat suppression in the MR scan may fail.

For example, in a field of view of a stomach of an obese patient, the substance composition ratio may indicate a high percentage of fat and a low percentage of water (adding up to 100%) so that the highest peak in the RFS will be the "fat peak". For another patient and/or another field of view, the highest peak may be, as is usually the case, the "water peak", as water is generally the dominant substance in the bodies of mammals.

One important piece of information for identifying peaks in the resonance frequency spectra, RFS, correctly is therefore information about the relative substance composition, RSC, of a patient's body in the field-of-view of a specific scan.

Said information about the relative substance composition, RSC, may also be used, or even be necessary, for certain shimmming procedures.

It is therefore one of the problems to be solved by the present invention to provide improved systems and methods for estimating a relative substance composition of a portion of a patient's body, in particular with respect to a more accurate and more precise estimation. This problem, as well as several related problems, are solved by the subject-matter of the independent claims. Advantageous refinements, variants and embodiments are described by the dependent claims as well as by the following description with reference to the drawings.

Thus, according to a first aspect of the present invention, a system is provided for estimating (or: determining) a relative substance composition, RSC, of a portion of a body of a patient in a field of view for a magnetic resonance image to be taken from the patient in a medical imaging scan, as defined in claim 1, the system comprising: an input interface for receiving at least one piece of patient information data, PPID, and for receiving at least one piece of field-of-view information data, PFID; a computing device configured to implement a trained machine learning algorithm, MLA, wherein the trained MLA is configured and trained to receive the at least one PPID and the at least one PFID received by the input interface as its input and to generate as its output at least one output signal indicating a relative substance composition, RSC, of a portion of the body of the patient for the medical image based on the at least one PPID and the at least one PFID; and an output interface for outputting at least the at least one output signal.

Instead of a fixed algorithm, a machine learning algorithm, MLA, is employed to generate the set of parameters of the relative substance composition, RSC, which has the advantage that the machine learning algorithm, MLA, can be continuously improved and updated.

Using the piece of patient information data, PPID, and the piece of field-of-view information data, PFID, enables the machine learning algorithm, MLA, to determine the relative substance composition, RSC, especially accurately and precisely.

The at least one piece of patient information data, PPID, and/or the at least one piece of field-of-view information data, PFID, may be realized as preferably one (but possibly also two or more) input vector for the machine learning algorithm, MLA, wherein each entry of said vector indicates a particular property of the patient or the field of view, respectively. Whenever herein a "vector" (a one-dimensional array) is mentioned, it shall be understood that this may equally refer to a matrix (a two-dimensional array) or a tensor (a three- or more-dimensional array), since any matrix or tensor may easily be "unfolded", i.e. reshaped into a vector structure. The input vector may in particular be used as an input vector for an artificial neural network, ANN, as one type of machine learning algorithm, MLA, wherein each entry of the input vector will be fed to one of the input nodes of the artificial neural network, ANN.

It shall be understood that the patient information data relate to (or: indicate, or: describe) the patient of which the medical image is to be taken with the field of view, and that the field-of-view information relates to (or: indicates, or: describes) the field of view with which the medical image is to be taken of the patient. In particular, the field of view may be understood to be a cuboid, the size of which is set within the B0 homogeneity area, for example to image a particular organ.

The input interface and/or the output interface may be realized in hardware and/or software. The input interface and/or the output interface can each comprise one or more different communication channels using one or more different communication protocols (e.g. HTTP). Each of the input interface and/or the output interface can be configured to connect to a cable-bound data connection and/or to a wireless data connection such as Bluetooth, Zigbee, WiFi and so on. The input interface and/or the output interface may also be configured to connect to Ethernet networks.

The computing device may be realized as any device, or any means, for computing. For example, the computing device may comprise at least one data processing (or: calculating) unit such as at least one central processing unit, CPU, and/or at least one graphics processing unit, GPU, and/or at least one field-programmable gate array, FPGA, and/or at least one application-specific integrated circuit, ASIC, and/or any combination of the foregoing. The computing device may comprise a working memory operatively coupled to the at least one processing unit and/or a non-transitory memory operatively connected to the at least one processing unit and/or the working memory.

The computing device may be partially or completely realized as a remote device, for example as a cloud computing platform.

According to the invention, the relative substance composition, RSC, comprises, or consists of, a ratio between water, fat, and at least one substance not naturally occurring in the human body from which a prosthesis for the human body may be fabricated, most preferred silicone. Other substances not naturally occurring in the human body may be surgical steel or any other substance from which a prosthesis for the human body may be fabricated.

In the most preferred variant the relative substance composition, RSC, consists of (or: describes, or: indicates) the relative percentages of water, fat, and silicone of the portion of the patient's body in the field of view for the medical image to be taken. Silicone is a substance frequently used for aesthetic or prosthetic reasons in the human body, for example as part of implants such as in a female breast or in a buttock.

The presence of non-naturally occurring substances such as silicone is often not evident even to a human operator and thus in the prior art the presence of such substances may have confused a human operator to some degree when interpreting resonance frequency spectra, RFS, in order to identify the peaks therein.

However, according to the present invention, the machine learning algorithm, MLA, is trained to accurately estimate (or: determine) the relative substance composition, RSC, which preferably includes a percentage for silicone and to output it in the output signal. In this case, in which the relative substance composition, RSC, comprises (or, more preferably, consists of, or in other words refers to) water, fat and silicone, it is strongly preferred that at least one piece of the at least one piece of patient information data, PPID, indicates information about implants in the body of the patient, preferably about amount, location and/or composition (i.e. substance used) of any implants.

For example, the at least one piece of patient information data, PPID, may comprise information for each of the most common locations for silicone implants (left breast, right breast, left buttock, right buttock and/or the like) about whether or not a silicone implant is located therein for the present patient. In an input vector, for example, each of said most common locations may be assigned one particular entry of the input vector, and a "0" may indicate the absence of a silicone implant in said location, whereas a "1" may indicate the presence of a silicone implant in said location. During training, the machine learning algorithm, MLA, will have learned that for fields of view which include such a location that is marked with a "1", it will usually be correct to estimate a non-zero percentage of silicone within the relative substance composition, RSC.

According to the invention, the at least one piece of patient information data, PPID, comprises at least one (and preferably all) of the following:
a) at least one a piece of information indicating the sex of the patient; for example, a particular binary numerical entry of an input vector may show "0" for biological female and "1" for biological male or vice versa.
b) at least one piece of information indicating at least one size or sizing of the patient; for example, a particular numerical entry of an input vector may indicate the patient's size in a given unit (centimeter, inch, ...) or a clothing sizing for at least one body part. Multiple entries of an input vector may indicate different sizes or sizings for different body parts.
c) at least one piece of information indicating a weight of the patient; for example, a particular numerical entry of an input vector may indicate the patient's weight in a given unit (kilograms, pounds, ...).
d) at least one piece of information indicating the age of the patient; for example, a particular numerical entry of an input vector may indicate the patient's age in a given unit (years, months, ...) or their birth date.
e) at least one piece of information indicating information about implants in the body of the patient; for example, as has been described in the foregoing, an input vector may comprise an entry for each of a predefined set of body regions, wherein each entry shows a "0" if no such silicone implant is present in the corresponding location and shows a "1" if there is a silicone implant present therein. Of course, for additional substances or types of implants (such as pacemakers, surgical steel replacements), additional entries of the input vector may be provided, preferably again one entry per body region per substance/type of implant.

According to the invention, the at least one piece of field-of-view information data, PFID, comprises at least one (and preferably all) of the following:
1) at least one piece of information indicating a field-of-view position; for example, one entry of an input vector may indicate an x-value, another entry a y-value, and a third entry a z-value in a given coordinate system;
2) at least one piece of information indicating a field-of-view size; and/or
3) at least one piece of information indicating a field-of-view rotation.

According to the invention, the input interface is further configured to receive at least one piece of information indicating a positioning of the patient (or: piece of patient positioning information, PPPI), and the trained MLA is further configured and trained to receive the at least one piece of patient positioning information, PPPI, as part of its input and to generate the at least one output signal also based on the at least one piece of patient positioning information, PPPI. The patient positioning shall in particular be understood to indicate how the body of the patient is positioned relative to the B0 homogeneity area and/or to indicate a table position of a table on which the patient rests. A particular organ to be imaged can be easily selected by choosing a combination of the field of view and the patient positioning.

The piece of patient positioning information, PPPI, may for example be a particular discrete numerical entry of an input vector which indicates a number of pre-defined positions, or may comprise a plurality of values indicating an absolute or relative patient positioning.

In some advantageous embodiments, refinements or variants of embodiments, the machine learning algorithm, MLA, is a feed-forward artificial neural network, ANN. The inventors have found that these types of artificial neural networks, ANN, are well suited for the present task. Preferably, the feed-forward ANN comprises an input layer with at least one input node for each of the pieces of information in the at least one PPID and/or at least one input node for each of the pieces of information in the at least one PFID.

In some advantageous embodiments, refinements or variants of embodiments, the feed-forward ANN comprises an output layer with at least two output nodes (preferably at least, or exactly, three output nodes) for generating the at least one output signal, wherein each output node outputs a (sub-)signal indicating an estimate for the percentage of a particular substance within the SMR. Two output nodes may indicate estimates for the percentages of water and fat; three output nodes may indicate estimates for the percentages of water, fat and silicone.

In some advantageous embodiments, refinements or variants of embodiments, the feed-forward ANN comprises between two and ten hidden layers with each in the range of from 32 to 5096 (both included) nodes (or: artificial neurons), preferably in the range from 128 to 2048 (both included), more preferably in the range from 256 to 1024 (both included). The inventors have found that feed-forward ANNs with these dimensions of layers are particularly well suited for the task at hand.

In some advantageous embodiments, refinements or variants of embodiments, after each of the hidden layers a drop-out function is applied, wherein the dropout rate of each dropout function is in the range of from 10% to 90% (both included), preferably in the range from 20% to 50% (both included).

In some especially preferred embodiments, the dropout rate is different in at least two of the applied drop-out functions. In some especially advantageous embodiments, the feed-forward ANN may comprise three hidden layers with (from layers closer to the input layer to layers closer to the output layer) 1024, 1024 and 256 neurons, respectively, wherein dropout functions with dropout rates of 20%, 50% and 40% being applied after every layer, respectively. In other words, after an input layer, a first hidden layer with 1024 nodes follows, thereafter a dropout function with 20% dropout rate, then a second hidden layer with 1024 nodes, thereafter a dropout function with 50% dropout rate, then a third hidden layer with 256 nodes, thereafter a dropout function with 40% dropout rate, and then the output layer.

According to a second aspect, the invention provides a method for estimating a relative substance composition, RSC, of a portion of a body of a patient in a field of view for a magnetic resonance image to be taken from the patient in a medical imaging scan, as defined in claim 7, said method comprising:
- receiving at least one piece of patient information data, PPID;
- receiving at least one piece of field-of-view information data, PFID;
- inputting the received at least one PPID and the received at least one PFID into a trained machine learning algorithm, MLA, wherein the trained MLA is trained to receive the at least one PPID and the at least one PFID as its input and to generate as its output at least one output signal indicating a relative substance composition, RSC, of the body of the patient for the medical image based on the at least one PPID and the at least one PFID; and
- outputting at least the at least one output signal.

Thus, also a method for using the system according to any embodiment of the first aspect of the present invention is provided.

In some advantageous embodiments, refinements, or variants of embodiments, the at least one output signal is used in a method for determining a water resonance setting frequency for an magnetic resonance imaging, MRI, scan. In this case, the invention also provides a method for determining a water resonance setting frequency for a magnetic resonance imaging, MRI, scan.

In some advantageous embodiments, refinements or variants of embodiments, the method includes receiving at least one piece of information indicating a positioning of the patient (or: piece of patient positioning information, PPPI), and inputting the at least one received PPPI into the trained MLA which is further configured and trained to receive the at least one piece of patient positioning information, PPPI, as part of its input and to generate the at least one output signal also based on the at least one piece of patient positioning information, PPPI.

According to a third aspect, a method for training a machine learning algorithm, MLA for use in the system according to any embodiment of the first aspect or for use in the method according to any embodiment of the second aspect is provided, the method comprising:
- providing training samples, each comprising a set of input parameters comprising at least one piece of patient information data, PPID, and at least one piece of field-of-view information data, PFID, in accordance with pieces of information which the machine learning algorithm, MLA, is configured to receive, and each labelled with a corresponding relative substance composition, RSC; and
- training the machine learning algorithm, MLA, with supervised learning using the provided training samples.

Training the machine learning algorithm, MLA, may comprise training the machine learning algorithm, MLA, by inputting the provided training samples into the machine learning algorithm, MLA, and by adjusting trainable (or: learnable) parameters of the machine learning algorithm, MLA, to minimize a loss function which penalizes differences between the relative substance composition, RSC, indicated by the at least one output signal based on each training sample and the corresponding relative substance composition, RSC, with which the corresponding set has been labelled.

According to a fourth aspect, a method for providing training samples for use in the method according to any embodiment of the third aspect of the present invention is provided, comprising:
- providing at least one family member of a virtual family, wherein each family member comprises a plurality of voxels for which the individual relative substance composition, RSC is known;
- virtually positioning each of the at least one family member in a plurality of positions and with a plurality of fields-of-view with regard to a medical imaging scan, optionally additionally with a plurality of table positions;
- determining, for each position and field-of-view for each family member of the virtual family, a total relative substance composition, RSC, for said field of view based on the individual RSCs of the voxels of said family member in said field of view at said position;
- generating sets of input parameters comprising at least one piece of patient information data, PPID, and at least one piece of field-of-view information data, PFID, based on the at least one family member, the plurality of positions and the plurality of fields-of-view; and
- labelling the generated sets with the corresponding determined total relative substance composition, RSC, to generate training samples.

In this way, a (sufficiently) large number of training samples can easily be generated.

According to a fifth aspect of the present invention, a computer program product as defined in claim 11 is provided comprising executable program code configured to, when executed by the computing device of the system according to the first aspect, perform the method according to any embodiment of the second aspect of the present invention and/or according to any embodiment of the third aspect of the present invention and/or according to any embodiment of the fourth aspect of the present invention.

According to a sixth aspect of the present invention, a non-transitory, computer-readable data storage medium as defined in claim 12 is provided, which comprises program code configured to, when executed by the computing device of the system according to the first aspect, perform the method according to any embodiment of the second aspect of the present invention and/or according to any embodiment of the third aspect of the present invention and/or according to any embodiment of the fourth aspect of the present invention.

Such a data storage medium may be, for example, a USB stick, a CD ROM, a DVD ROM, a Blue-Ray Disc, a hard drive, a solid state drive and/or the like.

According to an example outside of the scope of the present invention, a data stream is provided, which comprises program code (or which is configured to generate program code), configured to, when executed by a computing device, perform the method according to any embodiment of the second aspect of the present invention and/or according to any embodiment of the third aspect of the present invention and/or according to any embodiment of the fourth aspect of the present invention. The data stream may, for example, be provided by a server and be downloaded by a client or a user terminal or by a personal computer or a laptop.

Additional advantageous variants, refinements, embodiments and aspects of the invention will become more obvious in connection with the following description with reference to the drawings.

### Brief Description of the Drawings

The invention will be explained in greater detail with reference to exemplary embodiments depicted in the drawings as appended.

The accompanying drawings are included to provide a further understanding of the present invention and are incorporated in and constitute a part of the specification. The drawings illustrate the embodiments of the present invention and together with the description serve to illustrate the principles of the invention. Other embodiments of the present invention and many of the intended advantages of the present invention will be readily appreciated as they become better understood by reference to the following detailed description. Like reference numerals designate corresponding similar parts.

The numbering of method steps is intended to facilitate understanding and should not be construed, unless explicitly stated otherwise, or implicitly clear, to mean that the designated steps have to be performed according to the numbering of their reference signs. In particular, several or even all of the method steps may be performed simultaneously, in an overlapping way or sequentially.

In the drawings:
- Fig. 1: shows a schematic block diagram illustrating a system according to an embodiment of the first aspect of the present invention;
- Fig. 2: shows a schematic flow diagram illustrating a computer-implemented method according to an embodiment of the second aspect of the present invention;
- Fig. 3: shows a schematic flow diagram illustrating a computer-implemented method according to an embodiment of the third aspect of the present invention;
- Fig. 4: shows a schematic flow diagram illustrating a computer-implemented method according to an embodiment of the fourth aspect of the present invention;
- Fig. 5: shows a schematic block diagram illustrating a computer program product according to an embodiment of the fifth aspect of the present invention; and
- Fig. 6: shows a schematic block diagram illustrating a data storage medium according to an embodiment of the sixth aspect of the present invention;
- Fig. 7 and Fig. 8: illustrate typical resonance frequency spectra and the issues associated with them.

Although specific embodiments have been illustrated and described herein, it will be appreciated by those of ordinary skill in the art that the variety of alternate and/or equivalent implementations may be substituted for the specific embodiments shown and described without departing from the scope of the present invention defined by the appended claims. Generally, this application is intended to cover any adaptations or variations of the specific embodiments discussed herein.

### Detailed Description of the Invention

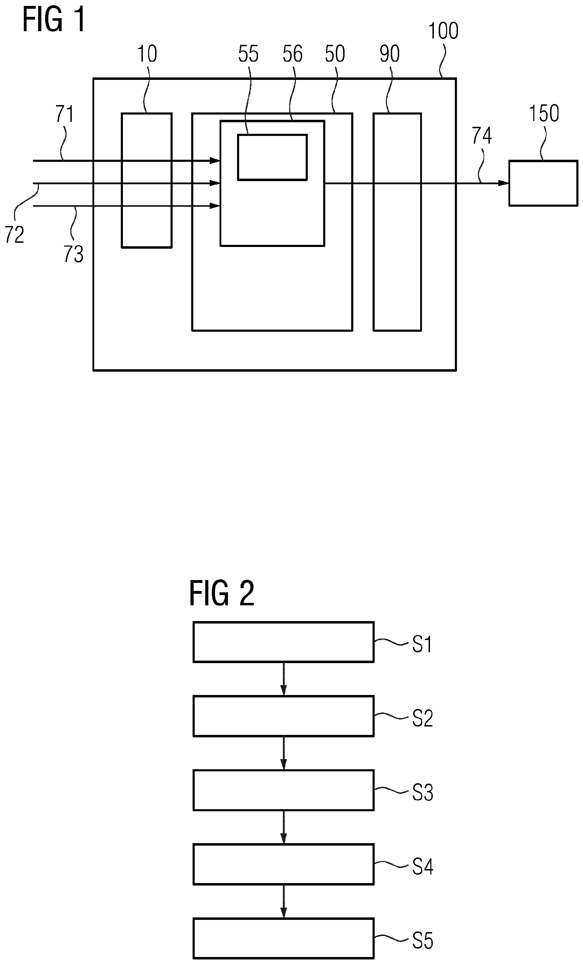

Fig. 1 shows a schematic block diagram illustrating a system 100 according to an embodiment of the first aspect of the present invention. Thus, Fig. 1 shows a schematic block diagram of a system 100 for estimating a relative substance composition, RSC, of a portion of a body of a patient in a field of view for a medical image to be taken from the patient in a medical imaging scan.

The system 100 comprises an input interface 10 for receiving at least one piece of patient information data, PPID 71, for receiving at least one piece of field-of-view information data, PFID 72 and for receiving at least one piece of patient positioning information, PPPI 73.

The at least one piece of patient information data, PPID 71, may comprise any or all of the pieces of information that have been discussed in the foregoing. The at least one piece of field-of-view information data, PFID 72, may comprise any or all of the pieces of information that have been discussed in the foregoing. The at least one piece of patient positioning information, PPPI 73, may for example be at least one piece of information indicating a positioning of the patient and/or indicating a table position of a table on which the patient rests.

The system 100 also comprises a computing device 50 configured to implement a trained machine learning algorithm, MLA 55. The computing device 50 may comprise a dedicated machine learning algorithm, MLA, module 56 configured to implement the trained MLA 55. The MLA module 56 may in particular be realized as software run by the computing device 50, wherein said software may be stored in a non-transitory data storage of the computing device 50 and may be run in a working memory of the computing device 50.

The trained MLA 55 is trained to receive the PPID 71, the PFID 72 and the PPPI 73 received by the input interface 10 as its input and to generate, as its output, based thereon at least one output signal 74. The at least one output signal 74 indicates an RSC of a portion of the body of the patient for the medical image, preferably a relative substance ratio between water, fat and silicone.

The system 100 further comprises an output interface 90 for outputting the generated at least one output signal 74. The generated at least one output signal 74 may be output e.g. to an external database for storing therein, or to any further device which makes use of the output signal 74, in particular of the information about the relative substance composition, RSC.

As is described elsewhere herein, the generated at least one output signal 74 may also be transmitted to a magnetic resonance imaging, MRI, device 150.

The MRI device 150 may be part of the system 100. For example, the input interface 10, the computing device 50 and the output interface 90 may be integrated into a housing of the device 150.

The MRI device 150 may be configured to perform a method for determining (and setting) a water resonance frequency setting based on the at least one output signal 74. For example, the MRI device 150 may be configured to perform a pre-scan, obtain thereby a resonance frequency spectrum, RFS, and to then determine, based on the RSF and on the RSC indicated by the at least one output signal 3, an optimal water resonance frequency setting (or analogue), and to set this optimal water resonance frequency setting for the MRI device 150.

In the following the case is described in detail in which the machine learning algorithm, MLA 55, is an artificial neural network, ANN, in particular a feed-forward artificial neural network.

The artificial neural network, ANN, comprises an input layer with at least one input node for each piece of patient information data, PPID 71, with at least one input node for each piece of field-of-view information data, PFID 72, and with at least one input node for each piece of patient positioning information, PPPI 73. For example, pieces of information of PPID 71, PFID 72 or PPPI 73 which indicate one of a plurality of classes may be realized as a one-hot vector, or as a single value indicating the class. For example, the PPID 71 regarding the sex of a patient may be realized as a one-hot vector wherein [1,0] indicates female and [0,1] indicates male, or it may be realized as a single value, wherein 0 indicates male, 1 indicates female, and 2 indicates diverse/neutral.

Thus, the input layer of the artificial neural network, ANN, may, as an example, comprise nodes for the following input information:
Nodes for pieces of patient information data, PPID 71, with (exemplary) possible values or dimensions for values in square brackets:
1x sex [0, 1, 2]
1x height [... m]
1x weight [... kg]
1x age [... years]
1x silicone implant in left breast [0, 1]
1x silicone implant in right breast [0, 1]
Nx silicone implant in ... [N possible body parts/sections wherein silicone implants are possible, leading to N additional input nodes]

Nodes for pieces of field-of-view information data, PFID 72, with dimensions of values in square brackets:
1x field of view position in X direction [... m]
1x field of view position in Y direction [... m]
1x field of view position in Z direction [... m]
1x field of view size in X direction [... m]
1x field of view size in Y direction [... m]
1x field of view size in Z direction [... m]
1x field of view rotation [...°]

Nodes for pieces of patient positioning information, PPPI 73, with dimensions of values in square brackets:
1x patient positioning [0, 1, 2, 3, 4, 5, 6, 7, 8, 9, ...]
1x table position in Z direction [... m]

Thus, in this example, the input layer of the feed-forwards ANN comprises 14+N input nodes.

The artificial neural network, ANN, further comprises an output layer with one node for each of the substances to be determined in the relative substance ratio, RSC. For example, in the case of water, fat and silicone, the output layer comprises three output nodes. The redundancy provided by providing explicit values for all three substances (rather than providing two, and calculating the third) is intended and can be used for a plausibility analysis of the results.

Between the input layer and the output layer, the artificial neural network, ANN, comprises at least one hidden layer. Preferably, the feed-forward ANN may comprises three hidden layers with (from layers closer to the input layer to layers closer to the output layer) 1024, 1024 and 256 neurons, respectively, wherein dropout functions with dropout rates of 20%, 50% and 40% being applied after every layer, respectively. In other words, after the input layer, a first hidden layer with 1024 nodes follows, thereafter a dropout function with 20% dropout rate, then a second hidden layer with 1024 nodes, thereafter a dropout function with 50% dropout rate, then a third hidden layer with 256 nodes, thereafter a dropout function with 40% dropout rate, and then the output layer follows.

Fig. 2 shows a schematic flow diagram schematically illustrating a computer-implemented method for estimating a relative substance composition, RSC, of a portion of a body of a patient in a field of view for a medical image to be taken from the patient in a medical imaging scan.

The method of Fig. 2 may be performed in particular with the system according to any embodiment of the first aspect of the present invention, preferably with the system as has been described with respect to Fig. 1. Thus, the method of Fig. 2 may be modified according to any modifications or variants of the system according to any embodiment of the first aspect and vice versa. Any or all of the method steps described in the following may also be performed simultaneously or essentially simultaneously.

In a step S1, at least one piece of patient information data, PPID 71, is received, e.g. via an input interface 10 as has been described in the foregoing.

In particular, any or all of the following may be received as pieces of patient information data, PPID 71:
a) at least one a piece of information indicating the sex of the patient; for example, a particular binary numerical entry of an input vector may show "0" for biological female and "1" for biological male or vice versa.
b) at least one piece of information indicating at least one size or sizing of the patient; for example, a particular numerical entry of an input vector may indicate the patient's size in a given unit (centimeter, inch, ...) or a clothing sizing for at least one body part. Multiple entries of an input vector may indicate different sizes or sizings for different body parts.
c) at least one piece of information indicating a weight of the patient; for example, a particular numerical entry of an input vector may indicate the patient's weight in a given unit (kilograms, pounds, ...) .
d) at least one piece of information indicating the age of the patient; for example, a particular numerical entry of an input vector may indicate the patient's age in a given unit (years, months, ...) .
e) at least one piece of information indicating information about implants in the body of the patient; for example, as has been described in the foregoing, an input vector may comprise an entry for each of a predefined set of body regions, wherein each entry shows a "0" if no such silicone implant is present in the corresponding location and shows a "1" if there is a silicone implant present therein. Of course, for additional substances or types of implants (such as pacemakers, surgical steel replacements, additional entries of the input vector may be provided, preferably again one entry per body region per substance/type of implant.

In a step S2, at least one piece of field-of-view information data, PFID 72, is received, e.g. via an input interface 10 as has been described in the foregoing.

In particular, any or all of the following may be received as pieces of field-of-view information data, PFID 72:
1) at least one piece of information indicating a field-of-view position; for example, one entry of an input vector may indicate an x-value, another entry a y-value, and a third entry a z-value in a given coordinate system;
2) at least one piece of information indicating a field-of-view size; and/or
3) at least one piece of information indicating a field-of-view rotation.

In an optional step S3, at least one piece of patient positioning information, PPPI 73, is received, for example at least one piece of information indicating a positioning of the patient and/or indicating a table position of a table on which the patient rests.

In a step S4, the received at least one PPID 71 and the received at least one PFID 72 (and optionally the at least one piece of patient positioning information, PPPI 73) are input into a trained machine learning algorithm, MLA 55, in particular into a trained feed-forward artificial neural network, ANN. The artificial neural network, ANN is configured and trained to receive the at least one PPID 71 and the at least one PFID 72 (and optionally the at least one PPPI 73) as its input and to generate as its output at least one output signal 74 indicating a relative substance composition, RSC, of the body of the patient for the medical image based on the at least one PPID 71 and the at least one PFID 72 (and optionally the at least one PPPI 73).

In a step S5, at least the at least one output signal 74 is output, for example via the output interface 90 as has been described in the foregoing.

In optional additional steps, the output signal 74 may be used in a method for determining a water resonance setting frequency for an magnetic resonance imaging, MRI, scan.

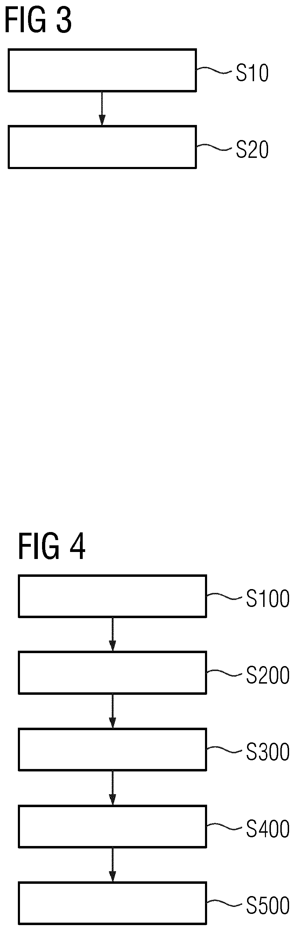

Fig. 3 shows a flow diagram schematically illustrating a computer-implemented method for training a machine learning algorithm, MLA, 55 in particular an artificial neural network, ANN, for use in a method or a system according to any embodiment of the first or the second aspect. In particular, the method illustrated with respect to Fig. 3 may be used for training a machine learning algorithm, MLA, 55 for use in a system 100 of Fig. 1 and/or for use with the method described with respect to Fig. 2.

In a step S10, (labelled) training samples are provided, each comprising a set of input parameters comprising at least one PPID 71 and at least one PFID 72 (and optionally at least one PPPI 73) in accordance with pieces of information which the MLA 55 is configured (and trained) to receive, and each labelled with a corresponding relative substance composition, RSC.

In a step S20, the MLA 55 is trained with supervised learning using the provided (labelled) training samples, for example using standard gradient propagation, known optimizers such as the ADAM optimizer and/or the like.

Fig. 4 shows a schematic flow diagram illustrating a method according to an embodiment of the fourth aspect of the invention, i.e. a method for providing training samples for use in the method according to any embodiment of the third aspect of the present invention, in particular for use in the method of Fig. 3.

In a step S100, at least one family member of a virtual family is provided, wherein each family member of the virtual family comprises a plurality of voxels for which the individual relative substance composition, RSC is known.

In a step S200, each of the at least one family member is virtually positioned in a plurality of positions with a plurality of fields-of-view with regard to a medical imaging scan, and optionally also with a plurality of table positions.

In a step S300, for each position and field-of-view (and optionally for each table position of the plurality of table positions) for each family member of the virtual family, a total relative substance composition, RSC, is determined for said field of view based on the individual RSCs of the voxels of said family member in said field of view at said position (and optionally with said table position).

In a step S400, sets of input parameters are generated which comprise at least one piece of patient information data, PPID 71, and at least one piece of field-of-view information data, PFID 72, (and optionally also at least one piece of patient positioning data, PPPI 73) based on the at least one family member, the (respective position out of the) plurality of positions and the (respective field of view out of the) plurality of fields-of-view (and optionally also on the respective table position out of the plurality of table positions).

In a step S500, the generated sets are labelled with the corresponding determined total relative substance composition, RSC, to generate training samples.

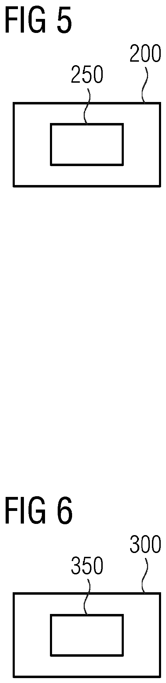

Fig. 5 shows a schematic block diagram illustrating a computer program product 200 according to an embodiment of the third aspect of the present invention. The computer program product 200 comprises executable program code 250 configured to, when executed by computing device 50 of system 100, to perform the method according to Fig. 2. Alternatively or additionally, the computer program product 200 may comprise executable program code 250 configured to, when executed by computing device 50 of system 100, to perform the method according to Fig. 3. Alternatively or additionally, the computer program product 200 may comprise executable program code 250 configured to, when executed by computing device 50 of system 100, to perform the method according to Fig. 4.

Fig. 6 shows a schematic block diagram illustrating a non-transitory, computer-readable data storage medium 300 comprising executable program code 350 configured to, when executed by computing device 50 of system 100, to perform the method according to Fig. 2. Alternatively or additionally, the data storage medium 300 may comprise executable program code 350 configured to, when executed by computing device 50 of system 100, to perform the method according to Fig. 3. Alternatively or additionally, the data storage medium 300 may comprise executable program code 350 configured to, when executed by computing device 50 of system 100, to perform the method according to Fig. 4.

In the foregoing detailed description, various features are grouped together in the examples with the purpose of streamlining the disclosure. It is to be understood that the above description is intended to be illustrative and not restrictive. It is intended to cover all alternatives, modifications and equivalence. Many other examples will be apparent to one skilled in the art upon reviewing the above specification, taking into account the various variations, modifications and options as described or suggested in the foregoing.

## Claims

1. A system (100) for estimating a relative substance composition, RSC, of a portion of a body of a patient in a field of view for a magnetic resonance image to be taken from the patient in a medical imaging scan, comprising:
an input interface (10) for receiving at least one piece of patient information data, PPID (71), and for receiving at least one piece of field-of-view information data, PFID (72);
a computing device (50) configured to implement a trained machine learning algorithm, MLA, (55) wherein the trained MLA (55) is configured and trained to receive the at least one PPID (71) and the at least one PFID (72) received by the input interface (10) as its input and to generate as its output at least one output signal (74) indicating an RSC of a portion of the body of the patient for the medical image based on the at least one PPID (71) and the at least one PFID (72); and
an output interface (90) for outputting at least the at least one output signal (74),
wherein the at least one PPID (71) comprises at least one of the following:
- at least one a piece of information indicating the sex of the patient;
- at least one piece of information indicating at least one size or sizing of the patient;
- at least one piece of information indicating a weight of the patient;
- at least one piece of information indicating the age of the patient and/or
- at least one piece of information indicating information about implants in the body of the patient,
wherein the at least one PFID (72) comprises at least one of the following:
- at least one piece of information indicating a field-of-view position;
- at least one piece of information indicating a field-of-view size;
- at least one piece of information indicating a field-of-view rotation,
**characterized in that** the RSC comprises, or consists of, a ratio between water, fat, and at least one substance not naturally occurring in the human body from which a prosthesis for the human body may be fabricated.

2. The system (100) of claim 1,
wherein the RSC comprises, or consists of, a ratio between water, fat, and silicone.

3. The system (100) of any of the claims 1 to 2,
wherein the at least one PPID (71) comprises, for each of a plurality of predefined body regions, a piece of information about whether or not said body region of the patient comprises a silicone implant.

4. The system (100) of any of claims 1 to 3,
wherein the input interface (10) is further configured to receive at least one piece of patient positioning information, PPPI (73); wherein the MLA (55) is configured and trained to also receive the at least one PPPI (73) as part of its input and to generate the output signal (74) based in addition also on the PPPI (73).

5. The system (100) of any of claims 1 to 4,
wherein the MLA (55) is a feed-forward artificial neural network, ANN, which comprises an input layer with at least one input node for each of the pieces of information in the at least one PPID (71) and/or at least one input node for each of the pieces of information in the at least one PFID (72).

6. The system (100) of claim 5,
wherein the feed-forward ANN comprises between two and ten hidden layers with each between 32 and 5096 nodes, wherein after each of the hidden layers a drop-out function is applied, wherein the dropout rate of each dropout function is between 10% and 90%.

7. A computer-implemented method for estimating a relative substance composition, RSC, of a portion of a body of a patient in a field of view for a magnetic resonance image to be taken from the patient in a medical imaging scan, comprising:
- receiving at least one piece of patient information data, PPID (71);
- receiving at least one piece of field-of-view information data, PFID (72);
- inputting the received at least one PPID (71) and the received at least one PFID (72) into a trained machine learning algorithm, MLA (55), wherein the trained MLA (55) is configured and trained to receive the at least one PPID (71) and the at least one PFID (72) as its input and to generate as its output at least one output signal (74) indicating a relative substance composition, RSC, of the body of the patient for the medical image based on the at least one PPID (71) and the at least one PFID (72); and
- outputting at least the at least one output signal (74),
wherein the at least one PPID (71) comprises at least one of the following:
- at least one a piece of information indicating the sex of the patient;
- at least one piece of information indicating at least one size or sizing of the patient;
- at least one piece of information indicating a weight of the patient;
- at least one piece of information indicating the age of the patient and/or
- at least one piece of information indicating information about implants in the body of the patient,
wherein the at least one PFID (72) comprises at least one of the following:
- at least one piece of information indicating a field-of-view position;
- at least one piece of information indicating a field-of-view size;
- at least one piece of information indicating a field-of-view rotation,
**characterized in that** RSC comprises, or consists of, a ratio between water, fat, and at least one substance not naturally occurring in the human body from which a prosthesis for the human body may be fabricated.

8. The method of claim 7, wherein the at least one output signal (74) is used in a method for determining a water resonance setting frequency for an magnetic resonance imaging, MRI, scan.

9. A computer-implemented method for training a machine learning algorithm, MLA (55) for use in the system of any of claims 1 to 6 or for use in the method of any of claims 7 or 8, comprising:
- providing training samples, each comprising a set of input parameters comprising at least one PPID (71) and at least one PFID (72) in accordance with pieces of information which the MLA (55) is configured to receive, and each labelled with a corresponding RSC; and
- training the MLA (55) with supervised learning using the provided training samples,
wherein the at least one PPID (71) comprises at least one of the following:
- at least one a piece of information indicating the sex of the patient;
- at least one piece of information indicating at least one size or sizing of the patient;
- at least one piece of information indicating a weight of the patient;
- at least one piece of information indicating the age of the patient and/or
at least one piece of information indicating information about implants in the body of the patient,
wherein the at least one PFID (72) comprises at least one of the following:
- at least one piece of information indicating a field-of-view position;
- at least one piece of information indicating a field-of-view size;
at least one piece of information indicating a field-of-view rotation.

10. A method of claim 9, wherein the providing of training samples comprises:
- providing at least one family member of a virtual family, wherein each family member comprises a plurality of voxels for which the individual RSC is known;
- virtually positioning each of the at least one family member in a plurality of positions and with a plurality of fields-of-view with regard to a medical imaging scan, optionally additionally with a plurality of table positions;
- determining, for each position and field-of-view for each family member of the virtual family, a total RSC for said field of view based on the individual RSCs of the voxels of said family member in said field of view at said position;
- generating sets of input parameters comprising at least one PPID (71) and at least one PFID (72) based on the at least one family member, the plurality of positions and the plurality of fields-of-view;
- labelling the generated sets with the corresponding determined total RSC to generate the training samples.

11. A computer program product (200) comprising executable program code (250) configured to, when executed on the computing device of the system of claim 1, perform the method according to any of claims 7 to 10.

12. A non-transitory, computer-readable data storage medium (300) ccomprising the computer program product of claim 12.

## Patentansprüche

1. System (100) zum Schätzen einer relativen Substanzzusammensetzung, RSC, eines Teils eines Körpers eines Patienten in einem Sichtfeld für ein Magnetresonanzbild, das von einem Patienten in einem medizinischen Bildgebungsscan aufgenommen werden soll, umfassend:
eine Eingabeschnittstelle (10) zum Empfangen mindestens eines Patienteninformationsdatenelements, PPID, (71) und zum Empfangen mindestens eines Sichtfeldinformationsdatenelements, PFID, (72) ;
eine Computervorrichtung (50), die zum Implementieren eines trainierten Algorithmus für maschinelles Lernen, MLA, (55) konfiguriert ist, wobei der trainierte MLA (55) konfiguriert und trainiert ist, um das mindestens eine PPID (71) und das mindestens eine PFID (72), die durch die Eingabeschnittstelle (10) empfangen werden, als seine Eingabe zu empfangen und mindestens ein Ausgangssignal (74), das eine RSC eines Teils des Körpers des Patienten für das medizinische Bild angibt, basierend auf dem mindestens einen PPID (71) und dem mindestens einen PFID (72) als seine Ausgabe zu erzeugen; und
eine Ausgabeschnittstelle (90) zum Ausgeben zumindest des mindestens einen Ausgangssignals (74),
wobei das mindestens eine PPID (71) mindestens eines von Folgenden umfasst:
- mindestens ein Informationselement, das das Geschlecht des Patienten angibt;
- mindestens ein Informationselement, das mindestens eine Größe oder Größeneinteilung des Patienten angibt;
- mindestens ein Informationselement, das ein Gewicht des Patienten angibt;
- mindestens ein Informationselement, das das Alter des Patienten angibt und/oder
- mindestens ein Informationselement, das Informationen über Implantate im Körper des Patienten angibt,
wobei das mindestens eine PFID (72) mindestens eines von Folgenden umfasst:
- mindestens ein Informationselement, das eine Sichtfeldposition angibt;
- mindestens ein Informationselement, das eine Sichtfeldgröße angibt;
- mindestens ein Informationselement, das eine Sichtfelddrehung angibt;
**dadurch gekennzeichnet, dass** die RSC ein Verhältnis zwischen Wasser, Fett und mindestens einer im menschlichen Körper nicht natürlich vorkommenden Substanz, aus der eine Prothese für den menschlichen Körper hergestellt sein kann, umfasst oder daraus besteht.

2. System (100) nach Anspruch 1,
wobei die RSC ein Verhältnis zwischen Wasser, Fett und Silikon umfasst oder daraus besteht.

3. System (100) einem der Ansprüche 1 bis 2,
wobei das mindestens eine PPID (71) für jede einer Mehrzahl von vordefinierten Körperregionen ein Informationselement darüber umfasst, ob die Körperregion des Patienten ein Silikonimplantat umfasst oder nicht.

4. System (100) einem der Ansprüche 1 bis 3,
wobei die Eingabeschnittstelle (10) ferner zum Empfangen mindestens eines Patientenpositionierungsinformationselements, PPPI, (73) konfiguriert ist, wobei der MLA (55) konfiguriert und trainiert ist, um außerdem das mindestens eine PPPI (73) als Teil seiner Eingabe zu empfangen und das Ausgangssignal (74) außerdem basierend auf dem PPPI (73) zu erzeugen.

5. System (100) einem der Ansprüche 1 bis 4,
wobei der MLA (55) ein künstliches neuronales Feedforward-Netzwerk, ANN, ist, das eine Eingabeschicht mit mindestens einem Eingabeknoten für jedes der Informationselemente in dem mindestens einen PPID (71) und/oder mindestens einem Eingabeknoten für jedes der Informationselemente in dem mindestens einen PFID (72) umfasst.

6. System (100) nach Anspruch 5,
wobei das Feedforward-ANN zwischen zwei und zehn verborgenen Schichten mit jeweils zwischen 32 und 5096 Knoten umfasst, wobei nach jeder der verborgenen Schichten eine Ausfallfunktion angewendet wird, wobei die Ausfallrate jeder Ausfallfunktion zwischen 10 % und 90 % liegt.

7. Computerimplementiertes Verfahren zur Schätzung einer relativen Substanzzusammensetzung, RSC, eines Teils eines Körpers eines Patienten in einem Sichtfeld für ein Magnetresonanzbild, das von einem Patienten in einem medizinischen Bildgebungsscan aufgenommen werden soll, umfassend:
- Empfangen mindestens eines Patienteninformationsdatenelements, PPID (71);
- Empfangen mindestens eines Sichtfeldinformationsdatenelements, PFID (72);
- Eingeben des empfangenen mindestens einen PPID (71) und des empfangenen mindestens einen PFID (72) in einen trainierten Algorithmus für maschinelles Lernen, MLA, (55), wobei der MLA (55) konfiguriert und trainiert wird, um das mindestens eine PPID (71) und das mindestens eine PFID (72) als seine Eingabe zu empfangen und mindestens ein Ausgangssignal (74), das eine relative Substanzzusammensetzung, RSC, des Körpers des Patienten für das medizinische Bild angibt, basierend auf dem mindestens einen PPID (71) und dem mindestens einen PFID (72) als seine Ausgabe zu erzeugen; und
- Ausgeben zumindest des mindestens einen Ausgangssignals (74),
wobei das mindestens eine PPID (71) mindestens eines von Folgenden umfasst:
- mindestens ein Informationselement, das das Geschlecht des Patienten angibt;
- mindestens ein Informationselement, das mindestens eine Größe oder Größeneinteilung des Patienten angibt;
- mindestens ein Informationselement, das ein Gewicht des Patienten angibt;
- mindestens ein Informationselement, das das Alter des Patienten angibt und/oder
- mindestens ein Informationselement, das Informationen über Implantate im Körper des Patienten angibt,
wobei das mindestens eine PFID (72) mindestens eines von Folgenden umfasst:
- mindestens ein Informationselement, das eine Sichtfeldposition angibt;
- mindestens ein Informationselement, das eine Sichtfeldgröße angibt;
- mindestens ein Informationselement, das eine Sichtfelddrehung angibt;
**dadurch gekennzeichnet, dass** die RSC ein Verhältnis zwischen Wasser, Fett und mindestens einer im menschlichen Körper nicht natürlich vorkommenden Substanz, aus der eine Prothese für den menschlichen Körper hergestellt sein kann, umfasst oder daraus besteht.

8. Verfahren nach Anspruch 7,
wobei das mindestens eine Ausgangssignal (74) in einem Verfahren zur Bestimmung einer Wasserresonanz-Einstellfrequenz für einen Magnetresonanzbildgebungs-,MRI-,Scan verwendet wird.

9. Computerimplementiertes Verfahren zum Trainieren eines Algorithmus für maschinelles Lernen, MLA, (55) zur Verwendung in dem System nach einem der Ansprüche 1 bis 6 oder zur Verwendung in dem Verfahren nach einem der Ansprüche 7 oder 8, umfassend:
- Bereitstellen von Trainingsmustern, die jeweils einen Satz von Eingabeparametern umfassen, der mindestens ein PPID (71) und mindestens ein PFID (72) gemäß Informationselementen umfasst, zu deren Empfang der MLA (55) konfiguriert ist, und die jeweils mit einer entsprechenden RSC gekennzeichnet sind; und
- Trainieren des MLA (55) mit überwachtem Lernen unter Verwendung der bereitgestellten Trainingsmuster,
wobei das mindestens eine PPID (71) mindestens eines von Folgenden umfasst:
- mindestens ein Informationselement, das das Geschlecht des Patienten angibt;
- mindestens ein Informationselement, das mindestens eine Größe oder Größeneinteilung des Patienten angibt;
- mindestens ein Informationselement, das ein Gewicht des Patienten angibt;
- mindestens ein Informationselement, das das Alter des Patienten angibt und/oder
mindestens ein Informationselement, das Informationen über Implantate im Körper des Patienten angibt,
wobei das mindestens eine PFID (72) mindestens eines von Folgenden umfasst:
- mindestens ein Informationselement, das eine Sichtfeldposition angibt;
- mindestens ein Informationselement, das eine Sichtfeldgröße angibt;
mindestens ein Informationselement, das eine Sichtfelddrehung angibt.

10. Verfahren nach Anspruch 9, wobei das Bereitstellen von Trainingsmustern umfasst:
- Bereitstellen mindestens eines Familienmitglieds einer virtuellen Familie, wobei jedes Familienmitglied eine Mehrzahl von Voxeln umfasst, für die die einzelne RSC bekannt ist;
- virtuelles Positionieren jedes des mindestens einen Familienmitglieds in einer Mehrzahl von Positionen und mit einer Mehrzahl von Sichtfeldern im Hinblick auf einen medizinischen Bildgebungsscan, optional zusätzlich mit einer Mehrzahl von Tischpositionen;
- Bestimmen für jede Position und jedes Sichtfeld für jedes Familienmitglied der virtuellen Familie einer Gesamt-RSC für das Sichtfeld basierend auf den einzelnen RSCs der Voxel des Familienmitglieds in dem Sichtfeld an der Position;
- Erstellen von Sätzen von Eingabeparametern, die mindestens ein PPID (71) und mindestens ein PFID (72) umfassen, basierend auf dem mindestens einen Familienmitglied, der Mehrzahl von Positionen und der Mehrzahl von Sichtfeldern;
- Kennzeichnen der erstellten Sätze mit der entsprechenden bestimmten Gesamt-RSC, um die Trainingsmuster zu erzeugen.

11. Computerprogrammprodukt (200), umfassend ausführbaren Programmcode (250), der so konfiguriert ist, dass er bei Ausführung auf der Computervorrichtung des Systems nach Anspruch 1 das Verfahren nach einem der Ansprüche 7 bis 10 durchführt.

12. Nichtflüchtiges computerlesbares Datenspeichermedium (300), umfassend das Computerprogrammprodukt nach Anspruch 12.

## Revendications

1. Système (100) d'estimation d'une composition de substance relative, RSC, d'une partie du corps d'un patient dans un champ de vue pour une image par résonance magnétique à prendre du patient dans un scan d'imagerie médicale, comprenant :
une interface (10) d'entrée de réception d'au moins un élément d'une donnée d'information de patient, PPID (71), et de réception d'au moins un élément d'une donnée d'information de champ de vue, PFID (72) ;
un dispositif (50) informatique configuré pour mettre en oeuvre un algorithme d'apprentissage machine, MLA (55), ayant subi un apprentissage, dans lequel le MLA (55) ayant subi un apprentissage est configuré et a subi un apprentissage pour recevoir la au moins une PPID (71) et la au moins une PFID (72) reçues par l'interface (10) d'entrée comme son entrée et pour créer à sa sortie au moins un signal (74) de sortie indiquant une RSC d'une partie du corps du patient pour l'image médicale reposant sur la au moins une PPID (71) et la au moins une PFID (72) et
une interface (90) de sortie pour sortir au moins un signal (74) de sortie,
dans lequel la au moins une PPID (71) comprend au moins l'un de ce qui suit :
- au moins un élément d'information indiquant le sexe du patient ;
- au moins un élément d'information indiquant au moins une taille ou un classement de taille du patient ;
- au moins un élément d'information indiquant un poids du patient ;
- au moins un élément d'information indiquant l'âge du patient et/ou
- au moins un élément d'information indiquant de l'information sur des implants dans le corps du patient,
dans lequel la au moins une PFID (72) comprend au moins l'un de ce qui suit :
- au moins un élément d'information indiquant une position du champ de vue ;
- au moins un élément d'information indiquant une dimension du champ de vue ;
- au moins un élément d'information indiquant une rotation du champ de vue ;
**caractérisé en ce que** la RSC comprend, ou consiste en, un rapport entre eau, graisse et au moins une substance ne se produisant pas naturellement dans le corps humain, en laquelle une prothèse pour le corps humain peut être fabriquée.

2. Système (100) suivant la revendication 1,
dans lequel la RSC comprend, ou consiste en, un rapport entre eau, graisse et silicone.

3. Système (100) suivant l'une quelconque des revendications 1 à 2,
dans lequel la au moins une PPID (71) comprend, pour chacune d'une pluralité de régions du corps définie à l'avance, un élément d'information sur le point de savoir si la dite région du corps du patient comprend un implant en silicone ou n'en comprend pas.

4. Système (100) suivant l'une quelconque des revendications 1 à 3,
dans lequel l'interface (10) d'entrée est configurée en outre pour recevoir un élément d'information de mise en position du patient, PPPI (73) ; dans lequel le MLA (55) est configuré et a subi un apprentissage pour recevoir également la au moins une PPPI (73) comme partie de son entrée et pour créer le signal (74) de sortie sur la base en outre de la PPPI (73).

5. Système (100) suivant l'une quelconque des revendications 1 à 4,
dans lequel le MLA (55) est un réseau neuronal artificiel à propagation vers l'avant, ANN, qui comprend une couche d'entrée, ayant au moins un noeud d'entrée pour chacun des éléments d'information dans la au moins une PPID (71) et/ou au moins un noeud d'entrée pour chacun des éléments d'information dans la au moins une PFID (72).

6. Système (100) suivant la revendication 5,
dans lequel l'ANN à propagation vers l'avant comprend entre deux et dix couches cachées ayant chacune entre 32 et 5096 noeuds, dans lequel après chacune des couches cachées une fonction de relâchement est appliquée, dans lequel le taux de relâchement de chaque fonction de relâchement est compris entre 10% et 90%.

7. Procédé mis en oeuvre par ordinateur d'estimation d'une composition de susbtance relative, RSC, d'une partie du corps d'un patient dans un champ de vue pour une image par résonance magnétique à prendre du patient dans un scan d'imagerie médicale, comprenant :
- recevoir au moins un élément d'une donnée d'information de patient PPID (71) ;
- recevoir au moins un élément d'une donnée d'information de champ de vue PFID (72) ;
- entrer la au moins une PPID (71) reçue et la au moins une PFID (72) reçue dans un algorithme d'apprentissage par machine ayant subi un apprentissage, MLA (55), dans lequel le MLA (55) ayant subi un apprentissage est configuré et a subi un apprentissage pour recevoir la au moins une PPID (71) et la au moins une PFID (72) à son entrée et pour créer à sa sortie au moins un signal (74) de sortie indiquant une composition de substance relative, RSC, du corps du patient pour l'image médicale sur la base de la au moins une PPID (71), de la au moins une PFID (72) et ;
- sortir au moins le au moins un signal (74) de sortie,
dans lequel la au moins une PPID (71) comprend au moins l'un de ce qui suit :
- au moins un élément d'information indiquant le sexe du patient ;
- au moins un élément d'information indiquant au moins une taille ou un classement de taille du patient ;
- au moins un élément d'information indiquant un poids du patient ;
- au moins un élément d'information indiquant l'âge du patient et/ou
- au moins un élément d'information indiquant de l'information sur des implants dans le corps du patient,
dans lequel la au moins une PFID (72) comprend au moins l'un de ce qui suit :
- au moins un élément d'information indiquant une position du champ de vue ;
- au moins un élément d'information indiquant une dimension du champ de vue ;
- au moins un élément d'information indiquant une rotation du champ de vue ;
**caractérisé en ce que** la RSC comprend, ou consiste en, un rapport entre eau, graisse et au moins une substance ne se produisant pas naturellement dans le corps humain, en laquelle une prothèse pour le corps humain peut être fabriquée.

8. Procédé suivant la revendication 7,
dans lequel on utilise le au moins un signal (74) de sortie dans un procédé de détermination d'une fréquence de réglage de la résonance de l'eau pour un scan d'imagerie par résonance magnétique, IRM.

9. Procédé mis en oeuvre par ordinateur pour faire l'apprentissage d'un algorithme d'apprentissage par machine, MLA (55) à utiliser dans le système suivant l'une quelconque des revendications 1 à 6, ou à utiliser dans le procédé suivant l'une quelconque de la revendication 7 ou 8, comprenant :
- on se procure des échantillons d'apprentissage comprenant chacun un ensemble de paramètres d'entrée comprenant au moins une PPID (71) et au moins une PFID (72) en accord avec des éléments d'information que le MLA (55) est configuré pour recevoir, et chacun étiqueté par une RSC correspondante et
- on fait subir un apprentissage au MLA (55) par un apprentissage supervisé utilisant les échantillons d'apprentissage procurés,
dans lequel la au moins une PPID (71) comprend au moins l'un de ce qui suit :
- au moins un élément d'information indiquant le sexe du patient ;
- au moins un élément d'information indiquant au moins une taille ou un classement de taille du patient ;
- au moins un élément d'information indiquant un poids du patient ;
- au moins un élément d'information indiquant l'âge du patient et/ou
au moins un élément d'information indiquant de l'information sur des implants dans le corps du patient,
dans lequel la au moins une PFID (72) comprend au moins l'un de ce qui suit :
- au moins un élément d'information indiquant une position du champ de vue ;
- au moins un élément d'information indiquant une dimension du champ de vue ;
au moins un élément d'information indiquant une rotation du champ de vue.

10. Procédé suivant la revendication 9, dans lequel se procurer les échantillons d'apprentissage comprend :
- se procurer au moins un élément d'une famille virtuelle, chaque élément de la famille comprenant une pluralité de voxels pour lesquels la RSC individuelle est connue ;
- mettre en position virtuellement chacun du au moins un élément de la famille dans une pluralité de positions et avec une pluralité de champs de vue eu égard d'un scan d'imagerie médical, éventuellement en outre par une pluralité de positions de table ;
- déterminer, pour chaque position et champ de vue pour chaque élément de la famille virtuelle, une RSC totale pour ledit champ de vue sur la base des RSCs individuelles des voxels dudit élément de la famille dans ledit champ de vue à ladite position ;
- créer des ensembles de paramètres d'entrée comprenant au moins une PPID (71) et au moins une PFID (72) sur la base du au moins un élément de la famille, de la pluralité de positions et de la pluralité de champs de vue ;
- étiqueter les ensembles créés par la RSC totale déterminée correspondante pour créer les échantillons d'apprentissage.

11. Produit (200) de programme d'ordinateur comprenant un code (250) de programme pouvant être exécuté et configuré pour, lorsqu'il est exécuté sur le dispositif informatique du système de la revendication 1, effectuer le procédé suivant l'une quelconque des revendications 7 à 10.

12. Support (300) de mise en mémoire non transitoire de données pouvant être déchiffré par ordinateur, comprenant le produit de programme d'ordinateur de la revendication 12.
